Europäisches Patentamt

European Patent Office

Office européen des brevets

(11) Publication number: **0 409 883 B1**

# EUROPEAN PATENT SPECIFICATION

(45) Date of publication of patent specification: 26.01.94

(51) Int. Cl.⁵: **C07K 15/00**, C12P 21/00, C12N 5/00, C12N 15/00, C07K 3/18, G01N 33/569, //(C12P21/00,C12R1:91)

(21) Application number: 89904818.5

(22) Date of filing: 12.04.89

(86) International application number: PCT/GB89/00386

(87) International publication number: WO 89/09790 (19.10.89 89/25)

(54) MONOCLONAL ANTIBODY TO ENTEROVIRUSES.

(30) Priority: 12.04.88 GB 8808586

(43) Date of publication of application: 30.01.91 Bulletin 91/05

(45) Publication of the grant of the patent: 26.01.94 Bulletin 94/04

(84) Designated Contracting States: DE FR GB IT

(56) References cited:
WO-A-83/03972

Biological abstracts, volume 86, n 1, 1988 (Philadelphia, PA, US), G.E. Yousef et al "Derivation and biochemical characterization of an enterovirus group-specific monoclonal antibody", see page AB-1241, abstract 10907 & Intervirology 28(3) : 163-170, 1987

(73) Proprietor: 3i RESEARCH EXPLOITATION LIMITED
91 Waterloo Road
London SE1 8XP(GB)

(72) Inventor: MOWBRAY, James Frederick
4 Branksome Way
New Malden, Surrey KT3 3AX(GB)
Inventor: YOUSEF, Mohamed Galal Eldin
18 Rowan Close, Armont Avenue
London W5(GB)

(74) Representative: Eyles, Christopher Thomas et al
W.P. THOMPSON & CO.
High Holborn House
52-54 High Holborn
London WC1V 6RY (GB)

EP 0 409 883 B1

Biological abstracts, volume 86, n 2, 1988 (Philadelphia, PA, US), G.E. Yousef et al "Clinical and research application of an enterovirus group-reactive monoclonal antibody", see page AB-1306, abstract 22384, & Intervirology 28(4) : 199-205, 1987 (1988)

Biological abstracts/Reports-Reviews-Meetings, volume 34, n 103316, 1988, G.E. Yousef et al "Lack of reactivity of hepatitis A virus with two entero virus group-specific reagents a monoclonal antibody and a complementary DNA probe" see title and terms, & Journal of Medical Microbiology (England) 1987, vol 24, n 4, pII

## Description

This invention relates to the use of a monoclonal antibody to enteroviruses in assays for antigens of enteroviruses and for antibodies to enteroviruses in sera and other liquid samples and to immunoassay kits comprising said antibody.

Enteroviruses are a group of viruses which proliferate in the gastrointestinal tract. These viruses are reported to contain single-stranded polycistronic RNA with positive polarity and to be approximately 27 nm in diameter. They exhibit cubic symmetry and have a buoyant density in CsCl of 1.34 g/ml, according to Andrews et al, "Viruses of vertebrates", 4th Edition, pp 1-37, Bailleric-Tindall, London (1978). Structurally the enteroviruses have four major virion polypeptides, VP1, VP2, VP3 and VP4. Enteroviruses are implicated in a variety of clinical conditions such as poliomyelitis, Bornholm disease, acute meningitis, acute myocarditis, diabetes mellitus type 1, orchitis and infections of the upper respiratory tract.

The group includes the polioviruses, Coxsackie A, Coxsackie B and the echoviruses. Some seventy-two distinct serotypes have been identified to date, with the recent addition of hepatitis A virus (HAV) to the group.

It has also been recently established that enterovirus infection is responsible for myalgic encephalomyelitis (ME), also known as "post-viral fatigue syndrome". Diagnosis of this infection has been hindered in the past because of the huge variety of enteroviruses for which no adequate diagnostic assay exists.

In the clinical situation, a separate characteristic assay is required for each of the various serotypes whereas, in practical terms, it is often the case that an assay for the group as a whole would suffice.

Enteroviruses are usually identified by neutralization employing intersecting antiserum pools using combinations so designed that each serum is incorporated in more than one pool. Thus identification is usually slow, expensive, and tedious.

It is known that a protein sequence, known as VP1, is partially conserved throughout the whole of the enterovirus group. However, although it is possible to raise antibodies to that sequence, it has not been possible to produce an antibody which binds to all the serotypes: all the previous antibodies bound to VP1 epitopes and to epitopes on other protein systems which were not conserved throughout the whole group.

The derivation and characterization of an enterovirus group-specific monoclonal antibody (designated 5-D8/1 antibody) is described in Intervirology 28:163-170 (1987). On page 165 of this paper experiments are described establishing the biological properties of the 5-D8/1 monoclonal antibody. The technique used required that a plate was incubated for 7 days at 37° in 5% $CO_2$ in air, the purpose of the incubation step being to permit equilibration, in other words to allow the antibody to compete with natural antibodies in the fluid under test for the epitopes on any VP1 protein present in the fluid.

The present invention seeks to improve methods for diagnosing enterovirus infections in patients and, in particular, to reduce significantly the time required for such diagnosis.

According to the present invention there is provided the use, for detecting by a surface bound immunoassay method an antigen of an enterovirus in a sample further containing human antibodies to said enterovirus, of a monoclonal antibody to a VP1 protein of an enterovirus, said monoclonal antibody having a paratope which is capable of binding to an epitope of the said VP1 protein, which epitope (i) is conserved throughout the group of enteroviruses (excluding hepatitis A virus) and (ii) is a different epitope from any epitope to which a human antibody binds.

This invention is based on the discovery that not only is the VP1 protein itself highly conserved throughout the group but that an epitope thereon is also conserved.

The monoclonal antibody used in this invention is capable of binding to the VP1 protein of an enterovirus (as herein defined) despite the presence of human antibodies to said VP1 protein. Preferably the hybridoma cell line used in the production of the monoclonal antibody is derived from a rodent, such as a mouse.

When tested against a wide range of enteroviral serotypes, the antibody used in this invention has been found to bind, with the notable exception of the hepatitis A virus. However, this clear evidence of significant genetic variation from other members of the group, along with other recent indications from other unconnected research, tends to suggest that the HAV virus has been incorrectly placed in the enterovirus group. Hence, in the context of the present specification, the definition "enterovirus" excludes the hepatitis A virus (HAV).

In a particularly preferred aspect of the present invention there is used the monoclonal antibody designated 5-D8/1 that is secreted by the hybridoma cell line deposited on 14th April 1988 at European Collection of Animal Cell Cultures, PHLS Centre for Applied Microbiology & Research, Porton Down, Salisbury SP4 OJG, U.K., under Accession Number 88041401.

Normally it will be convenient to provide the monoclonal antibody with an immunometric marker. Hence the invention also embraces use of a labelled antibody comprising a monoclonal antibody which binds to an epitope of a VP1 protein of an enterovirus, which epitope is conserved throughout the VP1 proteins of all enteroviruses (excluding hepatitis A virus), which antibody has been labelled with an immunometric marker. Such an immunometric marker may be a fluorogenic marker, a luminometric marker, an enzymatic marker, a hapten marker, or a radioisotope. Introduction of a marker into the monoclonal antibody can be effected using standard techniques. Fluorogenic markers are described in US-A-3940475. Luminometric markers include luminol. Enzymatic markers are described in US-A-3645090. Horseradish peroxidase is a suitable enzymatic marker and may be conjugated with the antibody with the aid of a cross-linking molecule, such as glutaraldehyde. Hapten markers include biotin. As examples of radioisotopes there can be mentioned the radioisotopes of iodine, such as $^{125}$I.

The invention further provides assays for enteroviruses, antigens thereof, and antibodies thereto in liquid samples such as sera and other biological fluids, utilising the said monoclonal antibody 5-D8/1.

A method for detecting the presence of an antigen of an enterovirus (other than hepatitis A virus) in a serum sample taken from a patient suspected of suffering from an infection caused by an enterovirus, which method comprises:

(a) incubating said serum sample with a monoclonal antibody to a VP1 protein of an enterovirus, said monoclonal antibody having a paratope which is capable of binding to an epitope of the said VP1 protein, which epitope (i) is conserved throughout the group of enteroviruses (excluding hepatitis A virus) and (ii) is a different epitope from any epitope to which a human antibody binds;
and then,
(b) without waiting for equilibration to occur, assaying the incubated sample for the presence of a complex containing the monoclonal antibody and the antigen.

In another aspect the invention provides a method of detecting the presence in a liquid sample taken from a patient of antibodies to an enterovirus which comprises:

(a) providing a liquid medium containing a first antibody which is a monoclonal antibody to a VP1 protein of an enterovirus that has a paratope which is capable of binding to an epitope of the said VP1 protein, which epitope (i) is conserved throughout the group of enteroviruses and (ii) is a different epitope from any epitope to which a human antibody binds;
(b) contacting the liquid sample with an immobilized second antibody which is selected from an anti-IgG and an anti-IgM;
(c) washing the thus contacted immobilized second antibody;
(d) contacting the washed immobilized second antibody with a VP1 protein of an enterovirus;
(e) subsequently contacting the VP1 protein-treated immobilized second antibody with the liquid medium of step (a); and
(f) monitoring the resulting antibody-treated immobilized second antibody for the presence or absence of bound first antibody, the presence of bound first antibody indicating the presence in the liquid sample of antibodies to an enterovirus. The liquid sample may be, for example, blood, serum, cerebral spinal fluid or a pericardial infusion. In this method a predetermined amount of the VP1 protein can be used in step (d), while a quantity of the liquid medium of step (a) is used in step (e) that contains a predetermined amount of the first antibody, so that the amount of bound first antibody observed in step (f) provides a measure of the level of antibodies to an enterovirus in the liquid sample.

We further provide a method of detecting an antigen of an enterovirus in a liquid sample which comprises:

(a) providing a first antibody which is a monoclonal antibody which is capable of binding to an epitope of a VP1 protein of an enterovirus, which epitope is conserved through the group of viruses known as enteroviruses and excluding hepatitis A virus; and is a different epitope from any epitope to which a human antibody binds;
(b) incubating a mixture of the liquid sample with a predetermined amount of the first antibody;
(c) providing a first substrate comprising an immobilised VP1 protein of an enterovirus;
(d) contacting the incubated mixture of step (b) with the first substrate of step (c);
(e) washing the thus contacted first substrate;
(f) providing a second substrate that comprises an immobilised VP1 protein of an enterovirus and that is substantially identical to the first substrate of step (c); and
(f) comparing the amount of bound first antibody on the washed first substrate of step (e) with a control sample obtained by contacting the second substrate of step (f) with a like predetermined amount of the first antibody to that used in step (b) and subsequently washing the thus contacted second substrate, a reduction in the amount of bound first antibody on the first substrate compared with the amount of bound substrate on the second substrate providing a measure of the enterovirus antigen level in the liquid sample.

An immunoassay kit may thus comprise in separate containers:

(1) a monoclonal antibody to a VP1 protein of an enterovirus that has a paratope which is capable of binding to an epitope of the said VP1 protein, which epitope (i) is conserved throughout the group of enteroviruses and (ii) is a different epitope from that to which a human antibody binds;

(2) an immobilised second antibody which is selected from an anti-IgG and an anti-IgM; and

(3) a VP1 protein of an enterovirus.

An alternative immunoassay kit comprises in separate containers:

(1) a monoclonal antibody which is capable of binding to an epitope of a VP1 protein of an enterovirus, which epitope is conserved throughout the group of viruses known as enteroviruses and excluding hepatitis A virus and is a different epitope from any epitope to which a human antibody binds; and

(2) a substrate comprising an immobilised VP1 protein of an enterovirus.

The various aspects of the present invention will now be described in the following description, by way of illustration.

The application of the monoclonal antibody 5-D8/1 in a diagnostic method will now be described.

## Identification of Clinical Isolates

In these tests, 130 different field isolates of enteroviruses were screened in duplicate by dot-blot enzyme-immunoassay by the method of Naz et al, Science, 225, 342-344 (1984). Eight clinical isolates of human adenovirus, two isolates of rotavirus, yellow fever virus and measles virus vaccine strains, as well as uninfected tissue culture cells, were included in the test as controls. After adsorption of the antigens on to strips of NC membrane, they were incubated first with 5-D8/1 antibody, and then with peroxidase-labelled rabbit anti-mouse gamma-globulin. The colour was then developed using diaminobenzidine and hydrogen peroxide, the strips were dried, and the colour density was read with a densitometer.

## Detection of Antigens in Circulating Immune Complexes

Sera were obtained from 87 patients with chronic post-viral fatigue syndrome. Another group of sera was obtained from 32 patients with other diseases associated with the formation of immune complexes, for example, subacute bacterial endocarditis, systemic lupus erythematosus, and rheumatoid arthritis. A third group of sera was obtained from 35 normal individuals matched for sex, age and geographical location. The second and third groups acted as controls. All of the sera in the first and second groups were found previously to have circulating IgM complexes, as measured by the polyethylene glycol precipitation technique of Burton-Kee et al [J. clin. Path. 33: pp 653-659 (1980)]. To study the nature of the complexed antigens a modification of the technique of Dambuyant et al. [Clin. exp. Path. 37: pp 424-432 (1979)] and of Zewdie [Ph.D. Thesis University of London (1983)] was used. Briefly, $10\mu l$ of undiluted peroxidase-conjugated 5-D8/1 antibody was added to $100\mu l$ of the patient's serum, and incubated for six days at 4°C in the presence of 0.02 mM EDTA. If the labelled antibody reacted with antigenic specificities in the IgM circulating immune complexes within the serum, then some of the labelled 5-D8/1 antibody became bound in the complex. After equilibration for six days, immune complexes were precipitated with polyethylene glycol at a final concentration of 2% in 10 mM EDTA. The amount of labelled antibody precipitated was then measured by adding substrate (4-aminophenazone and hydrogen peroxide) and measuring the colour spectrophotometrically.

## Immunohistochemical Staining

Immunofluorescence. Fixed frozen myocardial sections from a child who died of a proven Coxsackie B4 myocarditis were tested by the indirect immunofluorescence method of Gardner and McQuinn cited above. Acetone-fixed frozen sections were washed in phosphate-buffered saline and then incubated with a 1:100 dilution of the 5-D8/1 antibody ascitic fluid in a moist chamber for 1 hour at 37°C: this was followed by application of fluorescein isothiocyanate-conjugated rabbit anti-mouse globulin. After further incubation for one hour at 37°C, sections were counterstained with Evans blue and mounted in glycerol.

### Immunoperoxidase Staining

Formalin-fixed paraffin-embedded skeletal muscle autopsy material from CD-1 mice infected with Coxsackie virus B1 (Tucson stain), and Balb/C mice infected with Coxsackie virus A9 were tested by immunoperoxidase staining. Following dewaxing and rehydation, the staining was carried out by the technique of Norbert et al. Peroxidase activity was developed with diaminobenzidine and hydrogen peroxide by the method described by Graham et al [J. Histochem. Cytochem. 14: pp 291-302 (1966)]. Sections were then counterstained with haematoxylin and mounted in DPX™ mountant.

### RESULTS

### Identification of Clinical Isolates

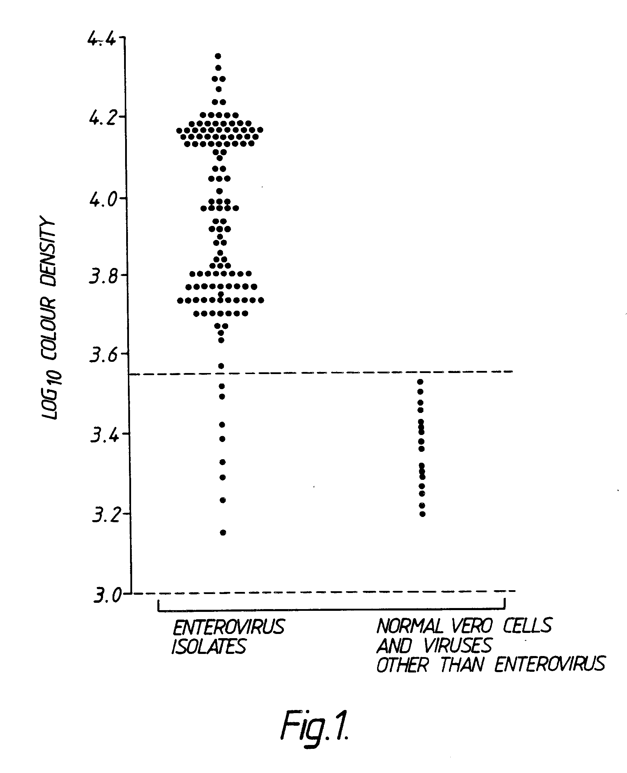

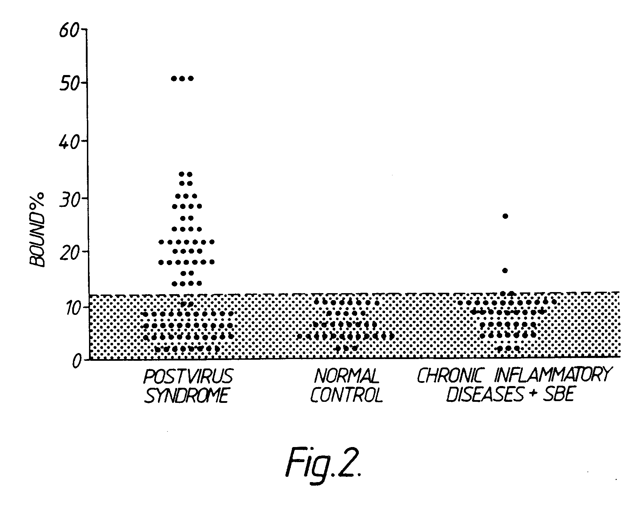

The results of identification by dot-blot enzyme immunoassays of the field isolates are shown in Figure 1. Each sample was run in duplicate, the mean value for each was calculated, and the $\log_{10}$ value of the mean plotted. The cutoff level for samples which were considered positive was taken as the mean of the control samples plus two standard deviations. It is evident from these results that 122 of the 130 clinical isolates (95%) were conveniently identified by the 5-D8/1 antibody as being enteroviruses. All of the control samples were negative in this test.

### Detection of Antigens in Circulating Immune Complexes

The cross-reactions between the sera of patients and controls with the peroxidase-labelled enterovirus group-reactive antibody 5-D8/1 are shown in Figure 2. Sera of patients with the post viral fatigue syndrome demonstrated a significant increase in binding with the labelled 5-D8/1 antibody, compared with the control groups ($x^2$ -24.1; $p <0.01$). This showed that the complexes in these patients contained antigens which were the same as, or cross-reactive with, the enterovirus epitope against which the 5-D8/1 monoclonal antibody is directed.

### In Situ Antigen Detection

By indirect immunofluorescence, the 5-D8/1 antibody selectively stained antigens in the post mortem heart sections obtained from a confirmed case of fatal acute myocarditis associated with Coxsackie virus B4 infection. Specificity was confirmed by using monoclonal antibody 14b in place of 5-D8/1. Immunofluorescence was confined to the cytoplasm of the individual myofibres, which is consistent with the cytoplasmic replication of the virus.

By indirect immunoperoxidase staining, 5-D8/1 gave prominent reactions with Coxsackie viruses B1 and A9 antigens in mouse tissues. Group-reactive antigens were still detectable after 5 weeks of infection in a section from a mouse which developed myositis after being infected with Coxsackie virus B1 (Tucson strain). The ability of the antibody 5-D8/1 to detect Coxsackie virus A9 antigens in a mouse which developed fatal myositis after virus inoculation was clearly demonstrated. Control sections stained with monoclonal antibody 14b in place of 5-D8/1 did not show any peroxidase activity.

### Discussion

The simple dot-blot enzyme immunoassay of this invention enabled 95% of field isolates to be identified. The remaining 5% of unidentified isolates were subsequently found to have low titers (< $10^4$ plaque-forming units/ml) and were identified by 5-D8/1 antibody by an indirect immunofluorescence assay. The results clearly demonstrate that the relevant VP1 epitope which is recognised by 5-D8/1 monoclonal antibody is highly conserved. The fact that assays conducted using the 5-D8/1 antibody do not identify the specific serotype is not believed to be of great clinical significance.

The prime benefit of methods employed in detection and measurement of circulating immune complexes has been in following disease activity in immune complex-associated diseases, and monitoring the effect of treatment on the prognosis of such diseases. These methods have not, however, provided any diagnostic help about the nature of the antigens responsible for the disease. The slow reaction technique employed in this invention utilises the simple principle of reversibility of antigen/antibody reactions at equilibrium. This technique enables the identification of specific viral antigens present in circulating complexes and thereby the identification of the infectious cause. The association of the enteroviruses with post-viral fatigue syndrome has been described [Behan et al. J. Infect. 105: pp 211-222 (1985) and McCartney et al. J. med. Virol. 19: pp 205-212 (1986)]. The test based upon monoclonal antibody 5-D8/1 show that about 50% of patients with this syndrome have IgM complexes which react strongly with the peroxidase-labelled 5-D8/1 antibody, indicating that the antigen in the complexes has specificity for the enterovirus group-reactive antibody.

There is little if any difficulty in staining fixed or frozen sections using either immunofluorescence or immunoperoxidase techniques. The group-reactive antibody 5-D8/1 makes an immunocytochemical approach to enterovirus studies a practical reality.

## Purification and labelling of 5-D8/1 antibody

A pool of 630 ml of ascitic fluid from mice bearing the hybridoma was collected 14 days after inoculation with the hybridoma intraperitoneally. Single radial immunodiffusion was carried out in 2mm gels containing 1.5% rabbit anti-mouse IgG antiserum in 1% agarose in 10mM EDTA in 0.15 sodium chloride. 10 $\mu$l of ascitic fluid was put in 4 mm wells, and purified mouse IgG standards in other wells. The plates were incubated overnight at 37°C, and the diameter of the rings measured the next day. The total amount of IgG in the pool was calculated to be 245 mg. An affinity column of 12 ml of protein A - Sepharose (Pharmacia) was prepared in a small 15 mm diameter column, using a 10 mM EDTA/0.15M sodium chloride buffer, pH 7.6. The ascitic fluid was dialysed against the same buffer, and applied to the column. After all the fluid had passed through the column, the column was washed in the buffer until the effluent did not contain detectable protein. The column was then eluted with 0.1M sodium citrate/citric acid buffer, pH 3.0, and the protein containing fraction collected, using a Pharmacia FPLC system. The eluate was concentrated by ultrafiltration, dialysed against 0.1M phosphate buffer, and the purity shown by immunoelectrophoresis against anti mouse serum. The final solution was stored in aliquots at -70°C until use.

## Radiolabelling.

10 $\mu$g of the affinity purified 5-D8/1 was labelled with $^{125}$I by the technique of Hunter & Greenwood ["Handbook of Immunological Methods", PP 17.2-17.12, ed Weir DM, Blackwell Scientific Publications, London (1974)] using 150$\mu$C of sodium iodide (Na[125]I). The reaction product was dialysed exhaustively against phosphate buffered saline, and stored at -70°C until use. The specific activity of the final preparation in a volume of 2 ml, was 9000cpm/$\mu$l, or 1.8 x 10$^6$ cpm per $\mu$g.

## Labelling with horse radish peroxidase

A two-stage method was used. 5 mg of horse radish peroxidase (HRP) was treated with a fourfold molar excess of redistilled glutaraldehyde, in 1.0 ml of 0.5M phosphate buffer pH 7.5. The free glutaraldehyde was removed by dialysis against phosphate buffered saline (PBS). The glutaraldehyde-HRP was added slowly to 12 mg of 5-D8/1 monoclonal antibody in 2 ml of PBS. The mixture was stirred at room temperature overnight. The remaining free glutaraldehyde groups were reacted with an excess of ethanolamine, and the final product dialysed against PBS. SDS PAGE electrophoresis showed that better than 95% of the HRP was bound to mouse IgG. The final solution was stored at -70°C until use.

## VP1 protein

VP1 protein was prepared by the method of Werner et al [DNA, Volume 7, No. 5, pp 307-316 (1988)].

## Immunoaffinity column

In order to purify VP1 preparations, a column of Sepharose 48 (Pharmacia) linked to the 5-D8/1 monoclonal antibody was prepared. A suspension of 25 ml of Sepharose 4B was cooled to 4°C after equilibration with 0.1M Na$_2$CO$_3$. Small fragments of solid cyanogen bromide were added, until no more would dissolve. During the addition the pH was monitored, and 1.0M Na$_2$CO$_3$ added to maintain the pH at or above pH 9.5. At the end of the reaction, the gel was washed, by decantation, with large amounts of water, and then resuspended in an approximately 20% v/v suspension in 0.1M phosphate buffer, pH 7.5. 17 mg of the 5-D8/1 monoclonal antibody in the same buffer was added, and the solution was stirred at 4°C for 6 hours. The gel was then washed with PBS and poured into a 15 mm diameter column.

4.5 ml of tissue culture supernatant of a 6 day culture of Vero cells inoculated with $10^5$ pfu Coxsackie B4 virus were heated at 56°C for 30 minutes, and treated with an equal volume of 0.05% sodium deoxycholate. The solution was clarified by centrifugation at 14,000g for 30 minutes. The supernatant was applied to the 5-D8/1 monoclonal antibody modified agarose column which was then washed with PBS until the effluent was free of protein. The column was then eluted with 0.1M citrate buffer pH 3.0 and 0.5 ml fractions collected.

The protein containing fractions were pooled and concentrated by ultrafiltration. 50 $\mu$l of the eluted fraction was electrophoresed in SDS PAGE, using BioRad™ electrophoresis equipment. Duplicate gels were prepared. One was stained with Aurodye™ for the protein bands, and the other transferred by electroelution to nitrocellulose membrane which was then blotted with HRP-labelled 5-D8/1 monoclonal antibody. There was one major band of 37 kd, with two very faint contaminant bands. Only the major band stained with HRP-labelled 5-D8/1 monoclonal antibody when counterstained with diaminobenzidine/hydrogen peroxide, indicating a high degree of purity of the eluted protein. One of the contaminant bands, with a molecular weight of 150 kd may represent small amounts of 5-D8/1 monoclonal antibody which were adsorbed, but not covalently linked to the column.

Immunohistochemical Staining for VP1 Antigen Detection

The monoclonal antibody 5-D8/1 can be used for detection of VP1 antigen in tissue samples by an immunohistochemical staining technique.

Tissue specimens

Tissue specimens of human origin were obtained as autopsy or biopsy material from patients with idiopathic polymyositis, PVFS, and acute myocarditis. Other tissue specimens were obtained from murine experimental models of polymyositis and myocarditis. Most of the samples were formalin-fixed and paraffin-embedded, and the rest were frozen.

Tissue samples which were fixed in buffered formalin (10% formalin in PBS at pH 7.4) were embedded in paraffin, cut 5 $\mu$m thick with a microtome blade and floated on a water bath. A pre-cleaned slide was thinly smeared with poly-1-lysine, as an adhesive, and the histologic sections were immediately picked up from the water bath. The excess water was drained and the slides were placed at 60°C for one hour, then stored away until used.

Cryostat sections (8$\mu$m) from frozen samples were mounted on coverslips and fixed for 20 minutes in cold acetone (-20°C) and kept frozen until used.

Indirect immunofluorescence (IF)

Cryostat sections of frozen samples were examined by immunofluorescence for the presence of enterovirus group-specific antigens as follows:

Sections were rehydrated in PBS and incubated in a 1:50 dilution of 5-D8/1 monoclonal Ab ascitic fluid in a moist chamber at room temperature for one hour. After washing in PBS for 20 minutes, FITC-conjugated rabbit anti-mouse gammaglobulin (1:50) was applied and incubated in a moist chamber for one hour at room temperature. Just prior to mounting of these tissues in glycerol, they were again washed in PBS (ten minutes), then in distilled water (one minute), and counterstained for five seconds in Evan's blue.

Indirect immunoperoxidase

The tissue sections were deparaffinized through three changes of xylol and rehydrated through graded ethanol (74% and 64%) and brought to distilled water. They were placed in a Coplin jar that contained 0.1% trypsin (Sigma) and 0.1% calcium chloride in distilled water with the pH of the solution adjusted to 7.8, and incubated for thirty minutes at 37°C. The sections were thoroughly washed in PBS and digested tissue were encircled with a wax pencil to confine reagents and sera. Thereafter, the following steps were performed:

a - Bleach acid haematin with 7.5% $H_2O_2$ in distilled water for five minutes. Wash off with tap water.

b - Inhibit endogenous peroxidase with 2.2% periodic acid in distilled water for five minutes. Wash off with tap water.

c - Block aldehyde groups with 0.02% potassium borohydride in distilled water for two minutes. Wash off with PBS (pH 7.2) containing 0.02% sodium azide.

d - Incubate in moist chamber at room temperature with first antibody (mouse ascitic fluid or serum) diluted in 1% ovalbumin in PBS for thirty minutes. Wash off with PBS.

e - Agitate in PBS bath for fifteen minutes.

f - Incubate with normal rabbit serum diluted 1:10, to block Fc receptors, for fifteen minutes. Wash off with PBS.

g - Incubate at room temperature with HRP-labelled rabbit anti-mouse gammaglobulin diluted 1:100 in PBS for thirty minutes. Wash off with PBS. The second antibody (enzyme-conjugated) is varied according to the species in which the first antibody was derived.

h - Agitate in PBS bath for fifteen minutes.

i - Incubate in diaminobenzidine, 5mg freshly dissolved in 10 ml 0.03% $H_2O_2$ in PBS. Wash with tap water as soon as brown pigments appear.

j - Counterstain with haematoxylin, dehydrate, and mount in DPX mountant.

### Results

The monoclonal antibody 5-D8/1, direct labelled with horse radish peroxidase, or used in indirect immunoenzyme and immunofluorescent techniques, was used to study a variety of tissues potentially infected with enteroviruses. 9/15 children with unexplained myocarditis and 7/10 cells from the cerebrospinal fluid of children with "viral meningitis" were positive. One childhood unidentified viral encephalitis was shown to be due to an enterovirus by staining a brain biopsy with 5-D8/1 antibody. In a study of chronic enterovirus infection of mice, samples of muscles obtained 5, 7, 14, 21, 28 and 35 days after infection showed specific staining for VP1 protein in each of 5 animals examined after each time period.

### Detection of viral antigen in tissue cultures

The monoclonal antibody 5-D8/1 can also be used for detection of viral antigen in tissue cultures.

### Indirect Immunofluorescence (IF)

The technique described by Gardner and McQuinn cited above was used with some modification. The antigen substrate for IF was prepared by spotting trypsin-dispersed cells, from infected and non-infected cell cultures, onto multispot glass slides, fixed in acetone for twenty minutes, and then dried and kept frozen until used. In the case of cerebrospinal fluid (CSF) specimens, cytospin preparations were fixed in acetone. The spots were exposed to undiluted or 1:10 diluted hybridoma supernatant for thirty minutes in a humid chamber at 37°C. After washing in PBS, the same spots were exposed to 1:40 diluted fluorescein isothiocyanate-conjugated (FITC) rabbit anti-mouse gammaglobulin diluted 1:40, for thirty minutes in a humid chamber at 37°C, washed in PBS, counterstained with Evan's blue and then mounted in glycerol. This technique was used to screen hybrids supernatants, detect viral antigens in field specimens, and study cross reactivity of monoclonal antibodies.

### Enzyme Immunobinding Assay (EIA)

A dot-blot EIA was performed with a modification of the technique described by Naz et al cited above. Briefly, a BioRad™ dot-blot apparatus was used to adsorb the antigens on strips of nitrocellulose (NC) membranes. Residual binding sites were saturated by blocking with 3% bovine serum albumin in 0.05 M tris, 0.15 M NaCl, 0.05% Tween 20™ (pH 10.3). The strips were incubated with monoclonal antibody 5-D8/1, and after appropriate washing they were incubated with a 1:200 dilution of peroxidase-conjugated rabbit anti-mouse gammaglobulin. The immunoblots were developed using 0.05% diaminobenzidine and 0.03% hydrogen peroxide in cold PBS, and colour density was scanned with a densitometer (Shimadzu - CS-930) and quantitated by integration of areas under peaks. This technique was applied to identify field isolates of enteroviruses, and to study cross-reactivity of the enterovirus group-specific monoclonal antibody 5-D8/1.

### Detection of viral antigen in tissue cultures

148 cultures of isolates of enteroviruses as wild strain isolates from the population, were cultured in Vero cells, and the cultures stained with 5-D8/1 antibody, by indirect immunofluorescence using a fluorescent anti-mouse IgG as the second antibody. In 145/148 the staining revealed strong fluorescence of infected cells in the culture, and in 25 control viral cultures no positives were seen. The results were obtained by an observer who did not know which viruses were in which culture. The identification of the enteroviral cultures was carried out on all 148 samples within one day, a result that could not be obtained by previously available methods.

Detection of VP1 in human blood samples

1. The first method involves addition of labelled antibody to serum, precipitating immune complexes, and measuring the fraction of labelled antibody in the precipitate.

In a case when the patient may have made antibody to the protein addition of labelled antibody to such serum may require a long time for equilibration to occur if the human and labelled added antibody have a similar specificity for epitopes of the viral antigen. (As we have subsequently found that the primate antibody and the mouse monoclonal antibody react with different epitopes, the time for equilibrium is not a problem. In case that were not so, an incubation time of 100 hours was used in this first detection method).

100$\mu$l of serum were mixed with 20 $\mu$g of peroxidase labelled 5-D8/1 monoclonal antibody, diluted in EDTA buffer, pH 7.6 in saline. The EDTA has a dual role, as buffer, and to prevent microbial growth during the incubation at 4°C for 100 hours. At the end of this time 500$\mu$l of 2.6% polyethylene glycol in EDTA saline was added, and after 4 hours at 4°C the precipitate was removed by centrifugation, and washed twice in 2 ml of 2% polyethylene glycol. The washed precipitate was redissolved in 500$\mu$l of EDTA saline and the samples transferred to 2 ml conical AutoAnalyzer™ cups. The peroxidase precipitated was measured in an AutoAnalyzer II™ using the standard phenol/aminophenazone technique, and hydrogen peroxide as substrate. The optical density was recorded and printed out as the percentage of the colour that would be produced if all the 5-D8/1 antibody had been precipitated. This was calculated from dilutions of the 5-D8/1 antibody which were run as standards in the assay. Samples from up to 10 normal healthy subjects were added in each assay run, and the mean and standard deviation for the values for the normal samples were calculated. Serum samples above the mean plus two standard deviations for the normal controls were considered positive, that is having detectable amounts of VP1 in the serum. It will be seen that the ability to detect enteroviral VP1 antigen is high, and the assay, although labour intensive, can be applied routinely to detect infection. Using one and a half technicians, it is possible to carry out assays at a rate of 13,000 assays per year.

2. The second method for measuring circulating VP1 antigen depends on the use of a surface bound immunoassay. Such a technique can only be successful if the human and mouse monoclonal antibodies in fact react with separate sites on VP1. This was carried out thus:

The antisera tested were from naturally immune human subjects, rabbits immunised with recombinant VP1 from Coxsackie B3, and monkeys hyperimmunised with 7 doses of Coxsackie B1 and B3 virus.

Polystyrene 2.5 ml tubes were coated with 1 ml of a 1:500 dilution of purified VP1 in a 0.1M carbonate/0.1M bicarbonate buffer pH 9.21 for 1 hour. The washed tubes were then blocked with a 1% solution of haemoglobin in phosphate buffered saline for 30 minutes at room temperature: 2$\mu$l of radiolabelled 5-D8/1 antibody, diluted in 1 ml PBS, were mixed with 10$\mu$l of serum and incubated for 30 minutes at 37°C. The radioactive 5-D8/1 antibody/serum mixtures were then added to the VP1-coated tubes and incubated at 37°C for 1 hour. After washing 3 times with PBS, the bound radioactivity was then measured in a gamma counter. The results are set out in Table I.

## Table I

### Inhibition of binding of radiolabelled 5-D8/1 antibody (*D8) to VP1 coated tubes with antisera of different species.

| Rabbit Anti-VP1 | Counts Bound (cpm) |
|---|---|
| 2μl *D8 | 3856, 2893 |
| (2μl *D8 + 2μl NRS) | 1890, 1787 |
| (2μl *D8 + 2μl Rabbit anti-VP1) | 4, 2 |
| (2μl *D8 + uncoated, but blocked tube) | 1, 4 |

| Primate Sera | Counts Bound |
|---|---|
| (*D8 + coated tube) | 3100, 2800 |
| (*D8 + monkey anti-B3) | 5800, 6100 |
| (*D8 + monkey anti-B1) | 6300, 5200 |
| (*D8 + Human antiserum 1) | 2700, 2100 |
| (*D8 + Human antiserum 2) | 2500, 2900 |

It will be seen from Table I that although immune rabbit serum produced marked inhibition of binding of the labelled 5-D8/1 antibody to the VP1, neither hyperimmune monkey serum, from animals immunised with three separate doses of enterovirus, nor adult human serum containing high levels of anti-enteroviral antibodies produced inhibition. In the case of the hyperimmune monkey sera the amount of binding actually increased. This result is explicable by the amount of rheumatoid factors present in such hyperimmune sera.

In neither human nor monkey sera was there any inhibition of binding of 5-D8/1 antibody, making it very likely that the two antibody specificities were for different epitopes on VP1. When rabbit, or mouse serum was used, however, there was dose dependent inhibition of binding, demonstrating that in this case the two antibodies were binding to the same, or very closely adjacent sites such that the antisera produce steric hindrance of the binding of the labelled 5-D8/1 antibody.

Having established that the human sera containing antibodies did not inhibit the binding of 5-D8/1 antibody to VP1 coated tubes, the effect of the addition of VP1 was studied. It will be seen that there is dose dependent inhibition of binding of the monoclonal antibody to the tubes, unaffected by the presence of human antibody. Thus this assay should be able to detect VP1 antigen, and not detect human antibody. This is only possible if the test antibody reacts with a site different from the epitope which is recognised by human antibodies, and 5-D8/1 is a suitable antibody for this purpose.

Attempts were then made to measure the amounts of VP1 present in human sera by inhibition of binding of 5-D8/1 antibody to the coated tubes. The results were standardised by the use of control, normal sera to which were added graded quantities of VP1. The inhibition obtained could then be expressed in terms of equivalent amounts of VP1 added to the control sera which could produce equivalent inhibition. The results are listed in Table II:

Table II

| Serum | Counts Bound |
|---|---|
| S1 alone | 6448, 6979 |
| S1 + 5$\mu$l VP1 | 2595, 3100 |
| S1 + 10$\mu$l VP1 | 1637, 1487 |
| S2 alone | 8129, 8149 |
| S2 + 2$\mu$l VP1 | 4055, 4830 |
| S2 + 5$\mu$l VP1 | 3008, 2109 |
| S2 + 10$\mu$l VP1 | 1634, 1291 |
| no serum, no VP1 | 9100, 8100 |

In the experiments summarised in Table II VP1 in serum was detected by inhibition of the binding of radiolabelled 5-D8/1 antibody to VP1 coated tubes. In each case the serum sample with known amounts of pure VP1 added was mixed with radiolabelled 5-D8/1 antibody and then added to the coated tube. Radioactivity bound after 30 minutes incubation was recorded. Serum S1 was positive in the first method for VP1, and contained about 1$\mu$l of VP1. The serum alone value is thus reduced below that seen with serum S2 which does not contain detectable amounts of VP1.

The conditions for optimisation of the coated tube assay were determined by measuring the amounts of labelled 5-D8/1 antibody bound when different doses were added to the tubes, and optimisation of the coating conditions. These results are shown in Tables III and IV:

Table III

| Effect of altering the concentration of VP1 used to coat tubes. | |
|---|---|
| Dilution of VP1 Solution | Counts Bound |
| Undiluted | 7540, 7752 |
| 1/10 | 4851, 4533 |
| 1/100 | 140, 73 |
| 1/1000 | 40, 18 |

In the tests of Table III 18000 cpm of radiolabelled 5-D8/1 antibody was used in each test.

Table IV

| Binding of radiolabelled 5-D8/1 antibody to optimally VP1 coated tube. | | |
|---|---|---|
| Amount of Antibody | Total Counts Added | Counts Bound |
| 1$\mu$l | 3900, 3685 | 574, 553 |
| 2$\mu$l | 8500, 8500 | 1732, 1128 |
| 3$\mu$l | 13555, 13546 | 3158, 2081 |
| 4$\mu$l | 17850, 18700 | 2010, 3569 |

These experiments illustrate the ability to use the monoclonal antibody to measure VP1 in human sera, uninfluenced by the presence of human antibodies to VP1, which are normally found in human sera (vide infra).

Detection of human and monkey anti-VP1 antibodies and immunoglobulin classes in serum, using sandwich ELISA/RIA techniques

The method used was a modification of the ELISA technique of El-Hagrassy et al [Lancet ii: pp 1159-1162 (1980)] replacing the polyclonal rabbit anti-enteroviral detector antibody with direct peroxidase-labelled 5-D8/1 antibody. The experiment then involves coating the microtiter plate with anti-human IgG or anti-human IgM antibody, adding the human sera to the plate, and binding the human immunoglobulin.

12

EP 0 409 883 B1

Enteroviral antibodies in the human immunoglobulin are able to capture antigens from an enteroviral preparation, and this in turn binds the detector antibody to measure the amount of viral antigen bound.

This test, using anti-human IgM, was carried out in duplicate on sera, with the detector antibody being either the polyvalent rabbit serum raised against Coxsackie B viruses, or the monoclonal antibody 5-D8/1. The results are set out in Table V below:

Table V

| Antigen | Adults and Children > 5 yrs | | Children > 5 yrs | |
|---|---|---|---|---|
| | Rabbit Polyclonal Antibody | 5-D8/1 Antibody | Rabbit Polyclonal Antibody | 5-D8/1 Antibody |
| CB1 | 14/14 | 14/14 | 6/6 | 2/6 |
| CB2 | 17/17 | 15/17 | 3/3 | 0/3 |
| CB3 | 15/15 | 15/15 | 5/5 | 2/5 |
| CB4 | 16/16 | 15/16 | 4/4 | 2/4 |
| CB5 | 18/18 | 18/18 | 2/2 | 2/4 |
| Sub Total | 80/80 | 77/80 | 20/20 | 8/20 |

As will be seen from Table V, in adult sera the 5-D8/1 antibody detected as many positives as the polyclonal antibody, but in young children there were examples when the standard assay identified children with monotypic responses to the serotype specific antigens. Some of the young children indicated to be positive by the standard serum are negative using 5-D8/1 monoclonal antibody as their antibodies are not directed against VP1 protein. In adults the heterotypic response against VP1 protein is present, so that "false negatives" are found largely in the children under 5 years old. It appears therefore that by the time exposure has been obtained to several enteroviruses (by age 5-6 years), all individuals are making antibody to the VP1 protein. The standard assay would miss some antibodies in people making immune responses to viruses separate from the serotypes overlapping Coxsackie B. More total positives are found using 5-D8/1 antibody than the polyclonal rabbit antiserum.

In this assay 5-D8/1 antibody can detect enteroviral infection, or even immunisation with poliovirus, and will be expected to detect all antibodies missed by the standard methods. The use of a standard reproducible reagent, rather than repeated production of dissimilar rabbit polyclonal antisera, must make for better standardisation of testing and consistency of results.

**Claims**

1. The use, for detecting by a surface bound immunoassay method an antigen of an enterovirus in a sample further containing human antibodies to said enterovirus, of a monoclonal antibody to a VP1 protein of an enterovirus, said monoclonal antibody having a paratope which is capable of binding to an epitope of the said VP1 protein, which epitope (i) is conserved throughout the group of enteroviruses (excluding hepatitis A virus) and (ii) is a different epitope from any epitope to which a human antibody binds.

2. The use, for detection by a surface bound immunoassay method of an antigen of an enterovirus in a serum sample taken from a patient suspected of suffering from an infection caused by an enterovirus and of having antibodies to said enterovirus in said serum sample, of a monoclonal antibody designated 5-D8/1 secreted by the hybridoma cell line deposited on 14th April 1988 at European Collection of Animal Cell Cultures under Accession No. 88041401.

3. A method of detecting the presence of an antigen of an enterovirus (other than hepatitis A virus) in a serum sample taken from a patient suspected of suffering from an infection caused by an enterovirus, which method comprises:
   (a) incubating said serum sample with a monoclonal antibody to a VP1 protein of an enterovirus, said monoclonal antibody having a paratope which is capable of binding to an epitope of the said VP1 protein, which epitope (i) is conserved throughout the group of enteroviruses (excluding hepatitis A virus) and (ii) is a different epitope from any epitope to which a human antibody binds;
   and then,

13

EP 0 409 883 B1

(b) without waiting for equilibration to occur, assaying the incubated sample for the presence of a complex containing the monoclonal antibody and the antigen.

4. A method according to claim 3, in which the monoclonal antibody is labelled with an immunometric marker.

5. A method according to claim 4, in which the immunometric marker is selected from fluorogenic markers, luminometric markers, enzymatic markers, hapten markers, and radioisotopes.

6. A method according to claim 5, in which the immunometric marker is horseradish peroxidase.

7. A method according to claim 5, in which the immunometric marker is radioactive iodine.

8. A method according to any one of claims 3 to 7, in which the monoclonal antibody is a monoclonal antibody designated 5-D8/1 secreted by the hybridoma cell line deposited on 14th April 1988 at European Collection of Animal Cell Cultures under Accession No. 88041401.

9. A method of detecting the presence in a liquid sample taken from a patient of antibodies to an enterovirus which comprises:
(a) providing a liquid medium containing a first antibody which is a monoclonal antibody to a VP1 protein of an enterovirus that has a paratope which is capable of binding to an epitope of the said VP1 protein, which epitope
(i) is conserved throughout the group of enteroviruses and
(ii) is a different epitope from any epitope to which a human antibody binds;
(b) contacting the liquid sample with an immobilised second antibody which is selected from an anti-IgG and an anti-IgM;
(c) washing the thus contacted immobilised second antibody;
(d) contacting the washed immobilised second antibody with a VP1 protein of an enterovirus;
(e) subsequently contacting the VP1 protein-treated immobilised second antibody with the liquid medium of step (a); and
(f) monitoring the resulting antibody-treated immobilised second antibody for the presence or absence of bound first antibody, the presence of bound first antibody indicating the presence in the liquid sample of antibodies to an enterovirus.

10. A method according to claim 9, in which the first antibody of step (a) is labelled with an immunometric marker.

11. A method according to claim 10, in which the immunometric marker is selected from fluorogenic markers, luminometric markers, enzymatic markers, hapten markers, and radioactive isotopes.

12. A method according to claim 10, in which the immunometric marker is horse radish peroxidase.

13. A method according to claim 10, in which the immunometric marker is a radioactive isotope of iodine.

14. A method according to claim 9, in which a predetermined amount of the VP1 protein is used in step (d), in which a quantity of the liquid medium of step (a) is used in step (e) that contains a predetermined amount of the first antibody, and in which the amount of bound first antibody observed in step (f) provides a measure of the level of antibodies to an enterovirus in the liquid sample.

15. A method according to any one of claims 9 to 14, in which the monoclonal antibody is a monoclonal antibody designated 5-D8/1 secreted by the hybridoma cell line deposited on 14th April 1988 at European Collection of Animal Cell Cultures under Accession No. 88041401.

16. A method of detecting an antigen of an enterovirus in a liquid sample which comprises:
(a) providing a first antibody which is a monoclonal antibody which is capable of binding to an epitope of a VP1 protein of an enterovirus, which epitope is conserved through the group of viruses known as enteroviruses and excluding hepatitis A virus; and is a different epitope from any epitope to which a human antibody binds;

14

(b) incubating a mixture of the liquid sample with a predetermined amount of the first antibody;

(c) providing a first substrate comprising an immobilised VP1 protein of an enterovirus;

(d) contacting the incubated mixture of step (b) with the first substrate of step (c);

(e) washing the thus contacted first substrate;

(f) providing a second substrate that comprises an immobilised VP1 protein of an enterovirus and that is substantially identical to the first substrate of step (c); and

(f) comparing the amount of bound first antibody on the washed first substrate of step (e) with a control sample obtained by contacting the second substrate of step (f) with a like predetermined amount of the first antibody to that used in step (b) and subsequently washing the thus contacted second substrate, a reduction in the amount of bound first antibody on the first substrate compared with the amount of bound substrate on the second substrate providing a measure of the enterovirus antigen level in the liquid sample.

17. A method according to claim 16, in which the first antibody of step (a) is labelled with an immunometric marker.

18. A method according to claim 17, in which the immunometric marker is selected from fluorogenic markers, luminometric markers, enzymatic markers, hapten markers, and radioactive isotopes.

19. A method according to claim 17, in which the immunometric marker is horse radish peroxidase.

20. A method according to claim 16, in which the immunometric marker is a radioactive isotope of iodine.

21. A method according to any one of claims 16 to 20, in which the monoclonal antibody is a monoclonal antibody designated 5-D8/1 secreted by the hybridoma cell line deposited on 14th April 1988 at European Collection of Animal Cell Culture under Accession No. 88041401.

22. An immunoassay kit comprising in separate containers:

(1) a monoclonal antibody to a VP1 protein of an enterovirus that has a paratope which is capable of binding to an epitope of the said VP1 protein, which epitope (i) is conserved throughout the group of enteroviruses and (ii) is a different epitope from any epitope to which a human antibody binds;

(2) an immobilised second antibody which is selected from an anti-IgG and an anti-IgM; and

(3) a VP1 protein of an enterovirus.

23. An immunoassay kit comprising in separate containers:

(1) a monoclonal antibody which is capable of binding to an epitope of a VP1 protein of an enterovirus, which epitope is conserved throughout the group of viruses known as enteroviruses and excluding hepatitis A virus and is a different epitope from any epitope to which a human antibody binds; and

(2) a substrate comprising an immobilised VP1 protein of an enterovirus.

**Patentansprüche**

1. Verwendung eines monoklonalen Antikörpers gegen ein VP1-Protein eines Entero-Virus, zum Nachweis eines Antigens eines Entero-Virus in einer Probe, die weiterhin menschliche Antikörper gegen diesen Entero-Virus enthält, mittels eines oberflächengebundenen Immunoessay-Verfahrens, wobei der monoklonale Antikörper ein Paratop aufweist, das zur Bindung an ein Epitop des VP1-Proteins fähig ist, wobei das Epitop (i) innerhalb der gesamten Gruppe der Entero-Viren (ausgenommen das Hepatitis-A-Virus) konserviert ist und (ii) ein anderes Epitop darstellt als solche Epitope, an die ein menschlicher Antikörper bindet.

2. Verwendung eines monoklonalen Antikörpers mit der Bezeichnung 5-D8/1, das durch die Hybridom-Zellinie, welche am 14. April 1988 bei der "European Collection of Animal Cell Cultures" unter der Hinterlegungsnummer 88041401 hinterlegt worden ist, ausgeschieden wird, zum Nachweis eines Antigens eines Entero-Virus in einer Serumprobe, die von einem Patienten stammt, der im Verdacht steht, an einer durch einen Entero-Virus verursachten Infektion zu leiden, und Antikörper gegen diesen Entero-Virus in dieser Serumprobe gebildet hat, mittels eines oberflächengebundenen Immunoessay-Verfahrens.

15

**3.** Verfahren zum Nachweis des Vorhandenseins eines Antigens von einem Entero-Virus (wobei der Hepatitits-A-Virus ausgenommen ist) in einer Serumprobe, die von einem Patienten stammt, der im Verdacht steht, an einer durch einen Entero-Virus verursachten Infektion zu leiden, wobei das Verfahren die Schritte umfaßt:

(a) Inkubierung der Serumprobe mit einem monoklonalen Antikörper gegen ein VP1-Protein eines Entero-Virus; wobei der monoklonale Antikörper ein Paratop aufweist, das zur Bindung an ein Epitop des VP1-Proteins geeignet ist, wobei das Epitop (i) in der gesamten Gruppe der Entero-Viren, ausgenommen das Hepatitis-A-Virus, konserviert ist und (ii) ein anderes Epitop darstellt als solche Epitope, an die ein menschlicher Antikörper bindet; und dann

(b) Durchführung eines Assays mit der inkubierten Probe zum Nachweis des Vorhandenseins eines Komplexes, der den monoklonalen Antikörper und das Antigen enthält, wobei nicht auf eine Einstellung des Gleichgewichts gewartet wird.

**4.** Verfahren gemäß Anspruch 3, worin der monoklonale Antikörper mit einem Immuno-Marker markiert ist.

**5.** Verfahren gemäß Anspruch 4, worin der Immuno-Marker aus fluorsszierenden Markern, lumineszierenden Markern, enzymatischen Markern, Hapten-Markern und Radioisotopen ausgewählt ist.

**6.** Verfahren gemäß Anspruch 5, worin der Immuno-Marker Meerrettich-Peroxidase ist.

**7.** Verfahren gemäß Anspruch 5, worin der Immuno-Marker radioaktives Jod ist.

**8.** Verfahren gemäß einem der Ansprüche 3 bis 7, worin der monoklonale Antikörper ein monoklonaler Antikörper mit der Bezeichnung 5-D8/1 ist, der durch die Hybridom-Zellinie ausgeschieden wird, die am 14. April 1988 bei der "European Collection of Animal Cell Cultures" unter der Hinterlegungsnummer 88041401 hinterlegt wurde.

**9.** Verfahren zum Nachweis des Vorhandenseins von Antikörpern gegen ein Entero-Virus in einer von einem Patienten stammenden flüssigen Probe, das die Schritte umfaßt:

(a) Bereitstellung eines flüssigen Mediums, das einen ersten Antikörper enthält, der ein monoklonaler Antikörper gegen ein VP1-Protein von einem Entero-Virus ist, der ein Paratop aufweist, welches zur Bindung an ein Epitop des VP1-Proteins fähig ist, wobei das Epitop (i) innerhalb der gesamten Gruppe der Entero-Viren konserviert ist, und (ii) ein anderes Epitop darstellt als solche Epitope, an die ein menschlicher Antikörper bindet;

(b) Inkontaktbringen der flüssigen Probe mit einem immobilisierten zweiten Antikörper, der aus einem Anti-IgG und einem Anti-IgM ausgewählt ist:

(c) Waschen des in dieser Weise in Kontakt gebrachten immobilisierten zweiten Antikörpers;

(d) Inkontaktbringen des gewaschenen immobilisierten zweiten Antikörpers mit einem VP1-Protein von einem Entero-Virus;

(e) anschließendes Inkontaktbringen des mit VP1-Protein behandelten, immobilisierten zweiten Antikörpers mit dem flüssigen Medium des Schritts (a); und

(f) Überwachen des erhaltenen, mit Antikörpern behandelten, immobilisierten zweiten Antikörpers auf Vorhandensein oder Nichtvorhandensein des gebundenen ersten Antikörpers, wobei das Vorhandensein des gebundenen ersten Antikörpers das Vorhandensein von Antikörpern gegen ein Entero-Virus in der flüssigen Probe anzeigt.

**10.** Verfahren gemäß Anspruch 9, worin der erste Antikörper den Schrittes (a) mit einem Immuno-Marker markiert ist.

**11.** Verfahren gemäß Anspruch 10, worin der Immuno-Marker ausgewählt ist aus Floureszenzmarkern, Lumineszensmarkern, enzymetrischen Markern, Hapten-Markern und radioaktiven Isotopen.

**12.** Verfahren gemäß Anspruch 10, worin der Immuno-Marker Meerrettich-Peroxidase ist.

**13.** Verfahren gemäß Anspruch 10, worin der immunometrische Marker ein radioaktives Isotop von Jod ist,

**14.** Verfahren gemäß Anspruch 9, worin eine vorbestimmte Menge des VP1-Proteins im Schritt (d) verwendet wird, worin eine Menge des flüssigen Mediums des Schritts (a) im Schritt (e) verwendet wird, das eine vorbestimmte Menge des ersten Antikörpers enthält, und worin die Menge des im Schritt (f) beobachteten gebundenen ersten Antikörpers einen meßbaren Gehalt an Antikörpern in der flüssigen Probe gegen den Entero-Virus angibt.

**15.** Verfahren gemäß einem jeden der Ansprüche 9 bis 14, worin der monoklonale Antikörper ain monoklonaler Antikörper mit der Bezeichnung 5-D8/1 ist, der durch die Hybridom-Zellinie ausgeschieden wird, die am 14. April 1988 bei der "European Collection of Animal Cell Cultures" unter der Hinterlegungsnummer 88041401 hinterlegt wurde.

**16.** Verfahren zum Nachweis einen Antigens eines Entero-Virus in einer flüssigen Probe, das die Schritte umfaßt:

(a) Bereitstellung eines ersten Antikörpers, der ein monoklonaler Antikörper ist, der zur Bindung an ein Epitop von einem VP1-Protein eines Entero-Virus fähig ist, wobei das Epitop innerhalb der gesamten Gruppe der Viren, die als Entero-Viren bekannt sind (ausgenommen das Hepatitis-A-Virus) konserviert ist; und ein anderes Epitop darstellt als solche Epitope, an die ein menschlicher Antikörper bindet;

(b) Inkubierung einer Mischung der flüssigen Probe mit einer vorbestimmten Menge des ersten Antikörpers;

(c) Bereitstellung eines ersten Substrats, das ein immobilisiertes VP1-Protein eines Entero-Virus umfaßt;

(d) Inkontaktbringen der inkubierten Mischung des Schritts (b) mit dem ersten Substrat des Schritts (c);

(e) Waschen des in dieser Weise in Kontakt gebrachten ersten Substrats;

(f) Bereitstellung eines zweiten Substrats, das ein immobilisiertes VP1-Protein von einem Entero-Virus umfaßt und im wesentlichen identisch ist mit dem ersten Substrat des Schritts (c); und

(g) Vergleichen der Menge des gebundenen ersten Anti-Körpers auf dem gewaschenen ersten Substrat des Schritts (e) mit einer Kontrollprobe, die beim Inkontaktbringen des zweiten Substrats des Schritts (f) mit einer gleichen vorbestimmten Menge des ersten, wie im Schritt (b) verwendeten Antikörpers erhalten wird und nachfolgendes Waschen das in dieser Weise in Kontakt gebrachten zweiten Substrats, wobei eine Reduzierung in der Menge des gebundenen ersten Antikörpers an das erste Substrat im Vergleich mit der Menge des gebundenen Substrats an das zweite Substrat ein Maß für den Gehalt an Antigen des Entero-Virus in der flüssigen Probe darstellt.

**17.** Verfahren gemäß Anspruch 16, worin der erste Antikörper den Schritts (a) mit einem Immuno-Marker markiert ist.

**18.** Verfahren gemäß Anspruch 17, worin der Immuno-Marker aus Floureszenzmarkern, Lumineszenzmarkern, enzymatischen Markern, Hapten-Markern und radioaktiven Isotopen ausgewählt ist.

**19.** Verfahren gemäß Anspruch 17, worin der Immuno-Marker Meerrettich-Peroxidase ist.

**20.** Verfahren gemäß Anspruch 16, worin der Immuno-Marker ein radioaktives Isotop des Jods ist.

**21.** Verfahren gemäß einem der Ansprüche 16 bis 20, worin der monoklonale Antikörper ein monoklonaler Antikörper mit der Bezeichnung 5-D8/1 ist, der durch die Hybridom-Zellinie ausgeschieden wird, die am 14. April 1988 bei der "European Collection of Animal Cell Cultures" unter der Hinterlegungssnummer 88041401 hinterlegt wurde.

**22.** Immuno-Assay-Kit, das in getrennten Behältern folgende Bestandteile umfaßt:

(1) einen monoklonalen Antikörper gegen ein VP1-Protein von einem Entero-Virus, der ein Paratop aufweist, das zur Bindung an ein Epitop des VP1-Proteins fähig ist, wobei das Epitop (i) innerhalb der gesamten Gruppe der Entero-Viren konserviert ist und (ii) ein anderes Epitop darstellt als solche Epitope, an die ein menschlicher Antikörper bindet;

(2) ein immobilisierter zweiter Antikörper, der aus einem Anti-IgG und einem Anti-IgM ausgewählt ist; und

(3) ein VP1-Protein von einem Entero-Virus.

**23.** Immuno-Assay-Kit, das in getrennten Behältern folgende Bestandteile umfaßt:

EP 0 409 883 B1

(1) einen monoklonalen Antikörper, der zur Bindung an ein Epitop von einem VP1-Protein von einem Entero-Virus fähig ist, wobei das Epitop innerhalb der gesamten Gruppe der Viren, die als Entero-Viren bekannt sind (ausgenommen das Hepatitis-A-Virus) konserviert ist und ein anderes Epitop darstellt als solche Epitope, an die ein menschlicher Antikörper bindet; und
(2) ein Substrat eines immobilisierten VP1-Proteins von einem Entero-Virus.

**Revendications**

1. Emploi, en vue de la détection par un procédé de dosage immunologique à liaison en surface d'un antigène d'un entérovirus dans un échantillon contenant en outre des anticorps humains dirigés contre ledit entérovirus, d'un anticorps monoclonal dirigé contre une protéine VP1 d'un entérovirus, ledit anticorps monoclonal ayant un paratope capable de se fixer à un épitope de ladite protéine VP1, lequel épitope (i) est constant dans tout le groupe des entérovirus (à l'exclusion du virus de l'hépatite A) et (ii) est un épitope différent de tous les épitopes auxquels se fixe un anticorps humain.

2. Emploi, en vue de la détection, par un procédé de dosage immunologique à liaison en surface, d'un antigène d'un entérovirus dans un échantillon de sérum prélevé chez un malade soupçonné de présenter une infection provoquée par un entérovirus et de posséder des anticorps dirigés contre ledit entérovirus dans ledit échantillon de sérum d'un anticorps monoclonal appelé 5-D8/1 sécrété par la lignée cellulaire d'hybridome déposée le 14 avril 1988 auprès de European Collection of Animal Cell Cultures sous le numéro 88041401.

3. Procédé de détection de la présence d'un antigène d'un entérovirus (autre que le virus de l'hépatite A) dans un échantillon de sérum prélevé chez un malade soupçonné de présenter une infection provoquée par un entérovirus, lequel procédé comprend:
   (a) l'incubation dudit échantillon de sérum avec un anticorps monoclonal dirigé contre une protéine VP1 d'un entérovirus, ledit anticorps monoclonal ayant un paratope capable de se fixer à un épitope de ladite protéine VP1, lequel épitope (i) est constant dans tout le groupe des entérovirus (à l'exclusion du virus de l'hépatite A) et (ii) est un épitope différent de tous les épitopes auxquels se fixe un anticorps humain;
   et puis
   (b) sans attendre la survenue de l'équilibre, le dosage de l'échantillon incubé pour détecter la présence d'un complexe contenant l'anticorps monoclonal et l'antigène.

4. Procédé selon la revendication 3, dans lequel l'anticorps monoclonal est marqué par un marqueur immunométrique.

5. Procédé selon la revendication 4, dis lequel le marqueur immunométrique est sélectionné parmi des marqueurs fluorogéniques, des marqueurs luminométriques, des marqueurs enzymatiques, des marqueurs de type haptènes et des radio-isotopes.

6. Procédé selon la revendication 5, dans lequel le marqueur immunométrique est de la peroxydase de raifort.

7. Procédé selon la revendication 5, dans lequel le marqueur immunométrique est de l'iode radioactif

8. Procédé selon l'une quelconque des revendications 3 à 7, dans lequel l'anticorps monoclonal est un anticorps monoclonal appelé 5-D8/1 sécrété par la lignée cellulaire d'hybridome déposée le 14 avril 1988 auprès de European Collection of Animal Cell Cultures sous le numéro 88041401.

9. Procédé de détection de la présence dans un échantillon de liquide prélevé chez un malade, d'anticorps dirigés contre un entérovirus, lequel comprend:
   (a) la mise à disposition d'un milieu liquide contenant un premier anticorps qui est un anticorps monoclonal dirigé contre une protéine VP1 d'un entérovirus et possède un paratope capable de se fixer à un épitope de ladite protéine VP1, lequel épitope (i) est constant dans tout le groupe des entérovirus et (ii) est un épitope différent de tous les épitopes auxquels se fixe un anticorps humain;
   (b) la mise en contact de l'échantillon de liquide avec un second anticorps immobilisé sélectionné parmi un anticorps anti-IgG et un anticorps anti-IgM;

18

(c) le lavage du second anticorps immobilisé ainsi mis en contact;

(d) la mise en contact du second anticorps immobilisé lavé avec une protéine VP1 d'un entérovirus;

(e) puis la mise en contact ensuite du second anticorps immobilisé traité par la protéine VP1 avec le milieu liquide de l'étape (a) ; et

(f) la surveillance du second anticorps immobilisé traité par un anticorps obtenu pour détecter la présence ou l'absence de premier anticorps fixé, la présence de premier anticorps fixé indiquant la présence dans l'échantillon de liquide d'anticorps dirigés contre un entérovirus.

10. Procédé selon la revendication 9, dans lequel le premier anticorps de l'étape (a) est marqué par un marqueur immunométrique.

11. Procédé selon la revendication 10, dans lequel le marqueur immunométrique est sélectionné parmi des marqueurs fluorogéniques, des marqueurs luminométriques, des marqueurs enzymatiques, des marqueurs de type haptènes et des isotopes radioactifs.

12. Procédé selon la revendication 10, dis lequel le marqueur immunométrique est de la peroxydase de raifort.

13. Procédé selon la revendication 10, dans lequel le marqueur immunométrique est un isotope radioactif de l'iode.

14. Procédé selon la revendication 9, dans lequel on utilise une quantité prédéterminée de protéine VP1 à l'étape (d), dans lequel on utilise une quantité prédéterminée du milieu liquide de l'étape (a) à l'étape (e), lequel contient une quantité prédéterminée de premier anticorps, et dans lequel la quantité de premier anticorps fixé observée à l'étape (f) donne une mesure de la concentration d'anticorps dirigés contre un entérovirus dans l'échantillon de liquide.

15. Procédé selon l'une quelconque des revendications 9 à 14, dans lequel l'anticorps monoclonal est un anticorps monoclonal appelé 5-D8/1 sécrété par la lignée cellulaire d'hybridome déposée le 14 avril 1988 auprès de European Collection of Animal Cell Cultures sous le numéro 88041401.

16. Procédé de détection d'un antigène d'un entérovirus dans un échantillon de liquide, lequel comprend:

(a) la mise à disposition d'un premier anticorps qui est un anticorps monoclonal capable de se fixer à un épitope d'une protéine VP1 d'un entérovirus, lequel épitope est constant dans le groupe des virus appelés entérovirus mais ne comprenant pas le virus de l'hépatite A ; et est un épitope différent de tous les épitopes auxquels se fixe un anticorps humain;

(b) l'incubation d'un mélange de l'échantillon de liquide avec une quantité prédéterminée de premier anticorps;

(c) la mise à disposition d'un premier substrat comprenant une protéine VP1 immobilisée d'un entérovirus;

(d) la mise en contact du mélange incubé de l'étape (b) avec le premier substrat de l'étape (c);

(e) le lavage du premier substrat ainsi mis en contact;

(f) la mise à disposition d'un second substrat comprenant une protéine VP1 immobilisée d'un entérovirus et qui est sensiblement identique au premier substrat de l'étape (c); et

(g) la comparaison de la quantité de premier anticorps fixé sur le premier substrat lavé de l'étape (e) avec un échantillon témoin obtenu par mise en contact du second substrat de l'étape (f) avec une quantité prédéterminée du premier anticorps identique à celle utilisée au cours de l'étape (b) et lavage subséquent du second substrat ainsi mis en contact, une infériorité de la quantité de premier anticorps fixé sur le premier substrat par comparaison à la quantité de premier anticorps fixé sur le second substrat donnant une mesure de la concentration d'antigène d'entérovirus dans l'échantillon de liquide.

17. Procédé selon la revendication 16, dans lequel le premier anticorps de l'étape (a) est marqué par un marqueur immunométrique.

18. Procédé selon la revendication 17, dans lequel le marqueur immunométrique est sélectionné parmi des marqueurs fluorogéniques, des marqueurs luminométriques, des marqueurs enzymatiques, des marqueurs de type haptènes et des isotopes radioactifs.

**19.** Procédé selon la revendication 17, dans lequel le marqueur immunométrique est de la peroxydase de raifort.

**20.** Procédé selon la revendication 16, dans lequel le marqueur immunométrique est un isotope radioactif de l'iode.

**21.** Procédé selon l'une quelconque des revendications 16 à 20, dans lequel l'anticorps monoclonal est un anticorps monoclonal appelé 5-D8/1 sécrété par la lignée cellulaire d'hybridome déposée le 14 avril 1988 auprès de European Collection of Animal Cell Cultures sous le numéro 88041401.

**22.** Kit de dosage immunologique comprenant, dans des récipients distincts:

(1) un anticorps monoclonal dirigé contre une protéine VP1 d'un entérovirus et possédant un paratope capable de se fixer à un épitope de ladite protéine VP1, lequel épitope (i) est constant dans tout le groupe des entérovirus et (ii) est un épitope différent de tous les épitopes auxquels se fixe un anticorps humain;

(2) un second anticorps immobilisé sélectionné parmi un anticorps anti-IgG et un anticorps anti-IgM; et

(3) une protéine VP1 d'un entérovirus.

**23.** Kit de dosage immunologique comprenant, dans des récipients distincts:

(1) un anticorps monoclonal capable de se fixer à un épitope d'une protéine VP1 d'un entérovirus, lequel épitope est constant dans tout le groupe des virus appelés entérovirus mais ne comprenant pas le virus de l'hépatite A et est un épitope différent de tous les épitopes auxquels se fixe un anticorps humain; et

(2) un substrat comprenant une protéine VP1 immobilisée d'un entérovirus.

*Fig.1.*

Fig.2.